# EUROPEAN PATENT APPLICATION

(11) **EP 4 670 653 A1**
(43) Date of publication of application: **31.12.2025**
(21) Application number: 24777680.0
(22) Date of filing: 08.03.2024
(51) Int. Cl.: A61B 17/221

(54) **RETRIEVAL STENT, RETRIEVAL DEVICE, AND RETRIEVAL SYSTEM**

(30) Priority: 28.03.2023 CN 202310316043
(71) Applicant: Shanghai MicroPort Medical (Group) Co., Ltd., Shanghai 201203 (CN)
(72) Inventor: GENG, Kangkang, Shanghai 201203 (CN); ZHOU, Qi, Shanghai 201203 (CN); WU, Jintian, Shanghai 201203 (CN); HUANG, Haiyong, Shanghai 201203 (CN)
(74) Representative: Patentship Patentanwaltsgesellschaft mbH
(86) International application number: PCT/CN2024/080679
(87) International publication number: WO 2024/198890

(57) **Abstract**

The present invention provides a retrieval stent, retrieval device and retrieval system. The retrieval system includes the retrieval device, a delivery device and an aspiration device. The delivery device is able to load the retrieval device and deliver it to a target lesion site. The aspiration device can be connected to the delivery device and is used to apply a suction force through the delivery device to aspirate an occlusive object from the target lesion site. The retrieval device includes a core tube and the retrieval stent. The retrieval stent is disposed over an outer circumference of the core tube at a distal end thereof, and the retrieval stent is attached to the core tube through a proximal loading section. The retrieval stent is self-expandable and fabricated from a mesh tube. A portion of the mesh tube is folded toward an exterior of the retrieval stent to form a distal curved section of the retrieval stent, and the proximal loading section of the retrieval stent is provided by a non-folded portion of the mesh tube. The distal curved section includes an umbrella-shaped mesh surface that is concave toward a proximal end. More efficient and better treatment of a thrombus, stone or other occlusive object can be achieved.

## Description

### TECHNICAL FIELD

The present invention relates to the field of medical devices, and more particularly to a retrieval stent, retrieval device and retrieval system.

### BACKGROUND

An embolism refers to the phenomenon where an insoluble abnormal material enters the circulating blood, flows with the blood, and subsequently blocks the lumen of a blood vessel. A pulmonary embolism (PE) is a blockage of a pulmonary artery or a branch thereof by a detached blood clot or other material, which impairs pulmonary circulation. PEs are characterized by high incidence, high mortality, high recurrence, frequent missed diagnosis, etc. After the pulmonary artery or branch thereof is blocked, the effects of mechanical blockage and neurohumoral factors would lead to increased pulmonary circulation resistance, higher pulmonary arterial pressure, and abnormal ventilation-perfusion ratio, which may in turn cause a series of changes including right heart failure, decreased systemic blood pressure, congestion and severe hypoxemia. In severe cases, pulmonary infarction, pulmonary collapse, impaired functions of the heart, brain, kidneys and other important organs, or even sudden death may occur.

Conventional treatment of thromboembolisms and/or PEs involves reducing and/or removing abnormal material mainly by, among others, pharmaceutical anticoagulation, surgery or minimally invasive intervention. Pharmaceutical anticoagulation is conservative and suitable for patients with mild symptoms. However, patients with severe symptoms, who have failed medication, have to undergo a necessary surgical procedure. Compared with traditional surgical treatment, minimally invasive intervention features minimal trauma and fast recovery and is therefore regarded as a very promising technique for the treatment of acute embolisms or PEs.

Minimally invasive intervention often employs approaches such as thrombolysis, rotational atherectomy, aspiration, basket, stent, or balloon thrombectomy to remove thrombus and other abnormal materials, thereby restoring blood flow within the vessel lumen. Among these approaches, stent or basket-based thrombectomy devices have been recognized by patients and surgeons and become an intensively studied topic of research in recent years because of excellent clinical outcomes. By establishing a sheath, a basket or other thrombectomy component may be advanced to a thrombus site inside a blood vessel. After that, it may be released so as to penetrate the blood clot and then withdrawn therewith out of the body through the access sheath. However, due to a lack of radial strength, conventional thrombectomy structure tends to deform when compressed by a thrombus, and during withdrawal, the thrombus tends to fall off the structure surface or escape through the openings therein, leading to low thrombus success and an increased risk of distal occlusion by disrupted thrombus. Moreover, the thrombus may fail to be carried away during withdrawal if it is hard thrombus. There are conventional thrombectomy structures in the form of distally-closed mesh structures, which are inadequately compliant and may collapse when used in a tortuous blood vessel, leading to an increased risk of escape or disruption of the captured thrombus. Moreover, due to a large contact area with the blood vessel, the blood vessel is more likely to be damaged. Further, it would be difficult for such structures to detach, capture and remove a thrombus that firmly adheres to a blood vessel wall. When used to remove a stone from a bile duct, ureter or the like, the conventional devices would present problems similar to those with thrombus removal.

### SUMMARY OF THE INVENTION

It is an object of the present invention to provide a retrieval stent, retrieval device and retrieval system, which overcome the problems associated with conventional treatment of thrombi, stones and other occlusive objects, including low treatment efficiency and success.

To this end, in a first aspect of the present invention, there is provided a retrieval stent, which is self-expandable and fabricated from a mesh tube and comprises a distal curved section and a proximal loading section. A portion of the mesh tube is folded toward an exterior of the mesh stent to form the distal curved section, wherein a non-folded portion of the mesh tube forms the proximal loading section, and wherein the distal curved section comprises an umbrella-shaped mesh surface that is concave toward a proximal end of the retrieval stent.

Optionally, a radial dimension of the distal curved section maybe greater than or equal to a diameter of a target lumen, and wherein a mesh opening density of the distal curved section gradually decreases from a center to a periphery.

Optionally, the retrieval stent may further comprise a graft provided over the distal curved section, wherein the graft covers a portion of the mesh openings in the distal curved section.

Optionally, the graft may be provided over a mesh opening-sparse region of the distal curved section.

Optionally, a radiopaque structure may be provided on the distal curved section, wherein the radiopaque structure is provided at a location of the distal curved section with a maximum radial dimension.

Optionally, the radiopaque structure may comprise an extension strut and a radiopaque object provided on the extension strut, wherein the extension strut is located within a mesh opening of the distal curved section, wherein one end of the extension strut is connected to a mesh strut, and wherein the other end of the extension strut is a free end.

Optionally, the distal curved section may comprise a proximal block surface and a distal block surface, wherein the proximal block surface and the distal block surface oppose each other along an axis of the retrieval stent, and wherein the proximal block surface is closer to the proximal end of the retrieval stent than the distal block surface.

Optionally, the distal block surface may be curved toward the proximal end of the retrieval stent, wherein the proximal block surface is curved toward the distal block surface, and wherein the proximal block surface comprises sparser mesh openings than the distal block surface.

Optionally, each of the proximal and distal block surfaces may comprise a curved shape or a polygonal shape.

Optionally, the proximal and distal block surfaces comprise a curved shape and are directly joined to each other, or wherein the distal block surface comprises a curved shape and the proximal block surface comprises a polygonal shape, that are directly joined to each other, or wherein the proximal and distal block surfaces comprise a polygonal shape and are connected by a curved transition mesh surface..

Optionally, the distal curved section may be outwardly folded and curved at a trailing end thereof that is away from the proximal loading section, thereby forming a non-invasive end, wherein a soft covering material is applied to an end face of the non-invasive end.

Optionally, the proximal loading section may be a straight tubular section, or wherein the proximal loading section comprises a plurality of bulge units that are radially raised outwardly, wherein the plurality of bulge units are arranged at an interval along an axis of the retrieval stent, and wherein adjacent bulge units are connected by a straight-extending connecting portion, and wherein the plurality of bulge units is integrally formed with the distal curved section.

Optionally, structures of the plurality of bulge units are same or different, and wherein the bulge unit comprises a shape of a sphere, a disc, a cylinder, a basket, a bag or a combination thereof.

Optionally, radial dimensions of the plurality of bulge units gradually increase or decrease from a distal end to the proximal end of the retrieval stent, and/or wherein mesh opening densities of the plurality of bulge units gradually decrease from the distal end to the proximal end of the retrieval stent.

Optionally, the bulge units may be proximally or distally deflectable.

To the above end, in a second aspect of the present invention, there is provided a retrieval device, comprising a core tube and the above described retrieval stent, wherein the retrieval stent is disposed over an outer circumference of the core tube at a distal end thereof, and wherein the retrieval stent is attached to the core tube though the proximal loading section.

Optionally, the retrieval device may comprise a plurality of retrieval stents, which are arranged at an interval along an axis of the core tube.

Optionally, mesh opening densities of the plurality of retrieval stents gradually decrease from the distal end to a proximal end of the core tube, and/or wherein radial dimensions of the plurality of retrieval stents gradually decrease or increase from the distal end to the proximal end of the core tube.

Optionally, a graft may be provided on the distal curved section, wherein the graft covers a portion of the mesh openings of the distal curved section, and wherein the graft is at least provided at a distalmost retrieval stent.

Optionally, a proximal end of the proximal loading section may be fixed to the core tube. Alternatively, each of the proximal end and a distal end of the proximal loading section is fixed to the core tube.

Optionally, the proximal loading section may be fixed to the core tube by radiopaque ring(s).

Optionally, the retrieval device may comprise a single retrieval stent, wherein the proximal loading section comprises a plurality of bulge units that are radially raised outwardly, wherein the plurality of bulge units are arranged at an interval along an axis of the retrieval stent, wherein the plurality of bulge units are integrally formed with the distal curved section, wherein a proximalmost bulge unit is fixed at a proximal end to the core tube and a proximal fixation point is formed, wherein a transitional connection between the distal curved section and a distalmost bulge unit is fixedly connected to the core tube and a distal fixation point is formed, and wherein a portion of the proximal loading section that is located between the proximal fixation point and the distal fixation point is disconnected from the core tube.

Optionally, the core tube may comprise a proximal stiff section and a distal elastic section, wherein the retrieval stent is disposed over the elastic section.

To the above end, in a third aspect of the present invention, there is provided a retrieval system, which comprises:
the retrieval device as defined above;
a delivery device configured to load and deliver the retrieval d device to a target lesion site; and
an aspiration device configured for connection with the delivery device and configured to apply a suction force through the delivery device to aspirate an occlusive object from the target lesion site.

As noted above, the present invention provides a retrieval stent which is self-expandable and fabricated from a mesh tube. A portion of the mesh tube is outwardly folded toward an exterior of the mesh stent to form a distal curved section of the retrieval stent, and a non-folded portion of the mesh tube provides a proximal loading section of the retrieval stent. The distal curved section comprises an umbrella-shaped mesh surface that is concave toward a proximal end of the retrieval stent.

With this arrangement, in the context of thrombectomy as an example, since the retrieval stent is made from a mesh tube, the distal curved section provided by an outwardly folded can provide sufficient radial support, which makes the retrieval stent more resistant to deformation when compressed by a thrombus and able to effectively disrupt and capture a thrombus during the thrombectomy procedure. Moreover, the captured thrombus will not easily fall off a surface of the retrieval stent or escape through one or more mesh openings thereof during withdrawal of the stent. In particular, a hard thrombus can be more easily carried out of the body due to the improved resistance of the retrieval stent to deformation. Apart from these, the retrieval stent is not closed and remains open at its trailing end. This imparts increased compliance, which makes the retrieval stent more resistant to collapse during advancement in a tortuous blood vessel and enables a smaller contact area of the distal curved section with a blood vessel, leading to less damage possibly caused to the blood vessel during removal of a thrombus therefrom. Further, the distal curved section can facilitate retraction of the retrieval stent into a delivery device during withdrawal. For these reasons, the retrieval stent provides higher thrombectomy efficiency and better thrombectomy performance than conventional thrombectomy structures.

As the device and system of the present invention are both based on the same inventive concept as the stent of the present invention and thus has all the advantages of the latter, the advantages thereof are not repeated herein.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 shows a front view of a retrieval system according to a first embodiment of the present invention;
Fig. 2a shows a front view of a retrieval device in a fully expanded configuration according to the first embodiment of the present invention;
Fig. 2b shows a schematic perspective view of the retrieval device in the fully expanded configuration according to the first embodiment of the present invention;
Fig. 3a shows a schematic perspective view of a retrieval stent in a fully expanded configuration according to the first embodiment of the present invention;
Fig. 3b shows a front view of the retrieval stent in the fully expanded configuration according to the first embodiment of the present invention;
Fig. 3c shows a schematic view of a distal end face of the retrieval stent in the fully expanded configuration according to the first embodiment of the present invention, as viewed in a distal-to-proximal direction;
Fig. 4a schematically illustrates a guide wire being advanced to a target lesion site during a surgical procedure according to the first embodiment of the present invention;
Fig. 4b schematically illustrates a sheath and a delivery catheter being successively advanced over the guide wire to a thrombus site during the surgical procedure according to the first embodiment of the present invention;
Figs. 4c to 4d schematically illustrate a plurality of retrieval stents being released as a result of retracting the delivery catheter during the surgical procedure according to the first embodiment of the present invention, in which the arrow indicates a direction of retraction;
Fig. 4e schematically illustrates the retrieval stents having all been completely released into a fully expanded configuration where they penetrate and cut into the thrombus during the surgical procedure according to the first embodiment of the present invention;
Fig. 4f schematically illustrates the retrieval device to remove the thrombus during the surgical procedure according to the first embodiment of the present invention;
Fig. 5 is a schematic representation of a retrieval stent with a graft according to a second embodiment of the present invention;
Fig. 6a is a schematic illustration of a retrieval stent containing a radiopaque structure according to a third embodiment of the present invention;
Fig. 6b is a schematic enlarged partial view of the retrieval stent of Fig. 6a;
Fig. 7 is a simplified schematic diagram of a retrieval stent according to a fourth embodiment of the present invention, which includes a distal curved section implemented as a combination of arched mesh surfaces curved in different directions;
Fig. 8 is a simplified schematic diagram of another retrieval stent according to the fourth embodiment of the present invention, which includes a distal curved section implemented as a combination of curved and polygonal mesh surfaces;
Fig. 9a is a simplified schematic diagram of yet another retrieval stent according to the fourth embodiment of the present invention, which includes a distal curved section comprising an outwardly folded non-invasive structure at one end thereof;
Fig. 9b is a schematic enlarged partial view of the retrieval stent of Fig. 9a;
Fig. 10 is a simplified schematic diagram of still yet another retrieval stent according to the fourth embodiment of the present invention, which includes a distal curved section implemented as a combination of polygonal mesh surface and a curved supporting mesh surface enabling a smaller contact area of the distal curved section with a luminal wall;
Fig. 11a schematically illustrates a retrieval device according to a fifth embodiment of the present invention, which includes multiple retrieval stents whose radial dimensions gradually decrease from distal end to proximal end;
Fig. 11b schematically illustrates a retrieval device according to a fifth embodiment of the present invention, which includes multiple retrieval stents whose radial dimensions gradually increase in the distal end to proximal end;
Fig. 12a is a schematic diagram showing the structure of a retrieval device according to a sixth embodiment of the present invention, which includes bulge units whose radial dimensions gradually decrease in a distal-to-proximal direction;
Fig. 12b is a schematic diagram showing the structure of another retrieval device according to the sixth embodiment of the present invention, which includes bulge units whose mesh opening densities gradually decrease in the distal-to-proximal direction;
Fig. 12c is a schematic diagram showing the structure of yet another retrieval device according to the sixth embodiment of the present invention, which includes a proximal bulge unit in the form of a basket; and
Fig. 13 is a schematic diagram showing the structure of a retrieval device according to a seventh embodiment of the present invention.

### List of Reference Numerals

100-retrieval device; 101-retrieval stent; 102-core tube; 103-distal curved section; 1031-mesh opening-dense region; 1032-mesh opening-sparse region; 1033-first mesh surface; 1034-second mesh surface; 1035-non-invasive end; 1036-soft covering material; 1037-transition mesh surface; 104-radiopaque ring; 105-proximal loading section; 1051-bulge unit; 1051a-projecting strut; 1051b-bag; 1052-connecting portion; 106-guide cap; 107 mesh opening in retrieval stent; 101a-trailing end; 101b-proximal end; 108-graft; 109-mesh strut; 110-radiopaque structure; 110a-extension strut; 110b-radiopaque object; 200-delivery device; 201-delivery catheter; 202-sheath; 202a-distal opening of sheath; 300-aspiration device; 301-aspiration tube; 302-coupling base; 303-sealing valve for preventing blood leakage; 304-one-way valve; 10-guide wire; 20-blood vessel; 30-thrombus.

### DETAILED DESCRIPTION

Objects, advantages and features of the present invention will become more apparent upon reading the following more detailed description with reference to the accompanying drawings, which illustrate particular embodiments thereof. Note that the figures are provided in a very simplified form not necessarily drawn to exact scale for the only purpose of helping to explain the disclosed embodiments in a more convenient and clearer way. In addition, the illustrated structures are usually part of their real-world counterparts. In particular, as the figures tend to have distinct emphases, they are sometimes drawn to different scales. Further, it is understood that, as used herein, the terms "first", "second", "third" and the like are only meant to distinguish various components, elements, steps, etc. from each other and are not intended to indicate logical or sequential orderings thereof, unless otherwise indicated or specified.

In the specification, "Distal end" usually refers to an end of the medical device that enters the body of a patient first in its normal operation, while "proximal end" usually refers to an end of the medical device closer to the operator in its normal operation. In the context herein, "distal end" and "proximal end" refer to relative locations, rather than exact endpoints. For instance, a "distal end" of a retrieval stent refers to a location near a distal endpoint thereof, rather than the distal endpoint. As used herein, the term "axial direction" usually refers to a direction along a central axis, e.g., of a retrieval stent, or of a core tube. "Radial direction" usually refers to a direction perpendicular to an axis. "Circumferential direction" refers to a direction about a central axis. As used herein, "target lumen" may refer to a lumen of a blood vessel, bile duct, ureter or the like. It should be notated that, as used herein, the term "radial dimension" refers to a length measured in a diametric direction, particularly in an expanded configuration. "Inner" refers to a location closer to a central axis, and "outer" refers to a location farther away from a central axis.

It is a principal object of the present invention to provide a retrieval stent, retrieval device and retrieval system, which overcome the problems associated with conventional treatment of thrombi, stones and other occlusive objects, including low treatment efficiency and success.

The present invention will be described below with reference to the accompanying drawings. In the following, should there be no conflict, the embodiments described hereunder and features thereof can complement or be combined with each other or one another.

### <Embodiment 1>

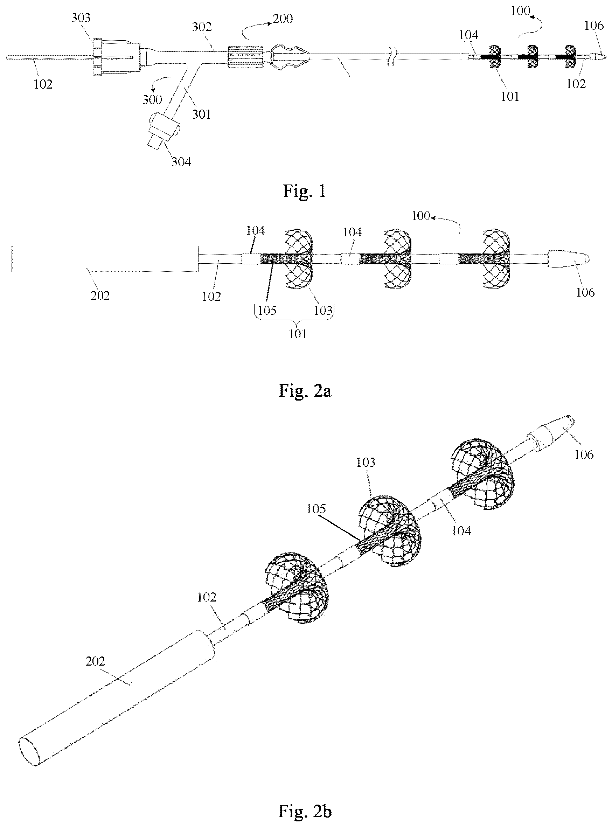

Now, a retrieval system according to a first embodiment of the present invention is described below with reference to Figs. 1, 2a and 2b, in the context of removing a thrombus from a blood vessel, as an example. As shown, the retrieval system includes a retrieval device 100, a delivery device 200 and an aspiration device 300. The retrieval device 100 includes a self-expanding retrieval stent 101. The retrieval stent 101 is fabricated by a mesh tube that is formed by cutting or braiding, then subjecting the mesh tube to a heat setting process to form the retrieval stent 101 with a desired configuration.

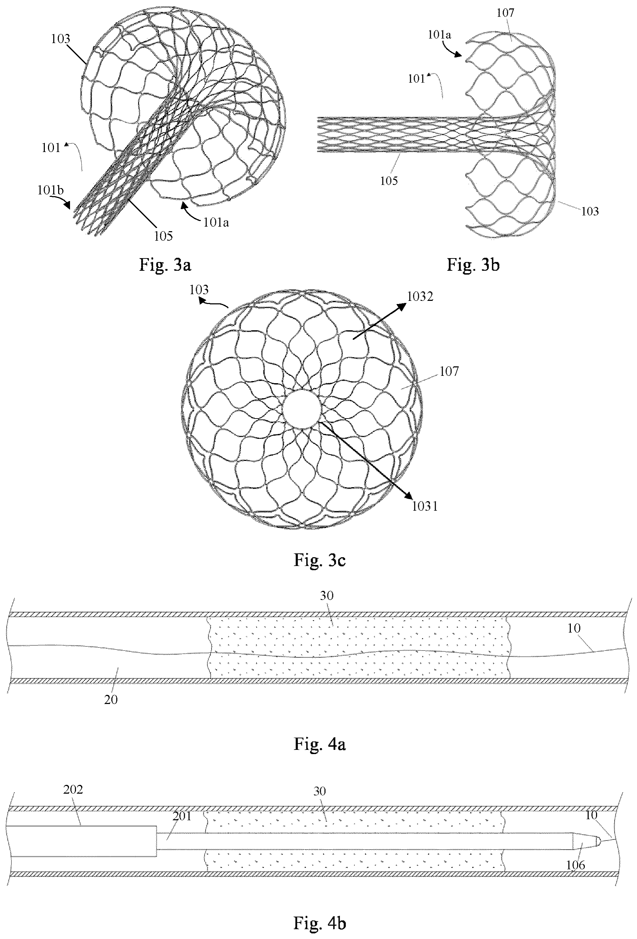

Referring to Figs. 3a and 3b, the stent 101 has a distal curved section 103 and a proximal loading section 105. In practice, a corresponding portion of the mesh tube may be folded toward an exterior of the mesh stent to form the distal curved section 103, with the remaining mesh tube that is not folded providing the proximal loading section 105. The proximal loading section 105 may be sleeved over an outer circumference of a core tube 102 around a distal end thereof, thereby mounting the retrieval stent 101 on the core tube 102 at a fixed location. The distal curved section 103 has an umbrella-shaped mesh surface, which is concave toward a proximal end of the retrieval stent 101. The shape of the umbrella-shaped mesh surface may be polygonal shape or curved shape, or combination of both polygonal shape and curved shape. Desirably, the distal curved section 103 and the proximal loading section 105 are connected by a smooth transition therebetween, that is a transition region between the distal curved section 103 and the proximal loading section 105 is smooth to prevent stress concentration. The transition that connects the distal curved section 103 and the proximal loading section 105 may be curved or linear in shape.

Referring back to Figs. 1, 2a and 2b, the retrieval device 100 further includes a core tube 102, and at least one retrieval stent 101 is disposed over an outer circumference of the core tube 102 around a distal end thereof. The retrieval stent 101 is mounted on the core tube 102 at a fixed location so that, in a fully expanded configuration of the retrieval stent 101, the umbrella-shaped mesh surface is curved toward a proximal end of the device 100 and therefore able to accommodate and to capture thrombus.

The number of the retrieval stents 101 may be multiple, and the multiple retrieval stents 101 may be sequentially arranged at an interval along an axis of the core tube 102, e.g., at equal or unequal intervals. The present invention is not limited to any particular number of retrieval stents 101. Although three retrieval stents 101 have been shown in the figures, in alternative embodiments, the retrieval device 100 may also include more or fewer retrieval stents 101 (e.g., one, two, four or more retrieval stents 101).

In a surgical procedure, the device 100 is delivered into the body of a patient with the aid of the delivery device 200. To this end, the delivery device 200 is used to load the retrieval device 100, the retrieval device 100 may be crimped into the delivery device 200 and carried thereby to a target lesion site. Of course, apart from delivering the retrieval device 100, the delivery device 200 may provide other functions, such as controlling release and withdrawal of the retrieval device 100. Those skilled in the art can understand the structure and functions of the delivery device 200 based on the knowledge in the art, and more detailed description thereof is omitted herein.

For more efficient and better thrombectomy, assistance from the aspiration device 300 is often necessary, which is provided by sucking a thrombus into the aspiration device 300 and extracting it out of the body. That is, the aspiration device 300 can be used to aspirate away a thrombus captured by the retrieval device 100. The aspiration device 300 may be coupled to the delivery device 200 either detachably or non-detachably. The aspiration device 300 is configured to apply a suction force through the delivery device 100 to suck a thrombus away from a target lesion site. Similarly, those skilled in the art can understand the structure and functions of the aspiration device 300 based on the knowledge in the art, and more detailed description thereof is omitted herein.

It will be understood that, differing from conventional thrombectomy structures, the retrieval stent 101 is formed from a mesh tube in accordance with present embodiment and therefore exhibits enhanced radial strength, which makes the retrieval stent 101 more resistant to deformation caused by compression by a thrombus during thrombectomy and enables it to effectively disrupt and capture a thrombus. Moreover, during withdrawal, the captured thrombus is less likely to fall off a surface of the retrieval stent 101 or escape though one or more mesh openings 107 thereof. Thus, not only more efficient thrombus capture can be achieved, but the risk of distal thrombotic vascular occlusion can be lowered. In particular, the increased resistance to deformation allows the retrieval stent 101 to more easily carry a hard thrombus during withdrawal out of the body, thereby resulting in increased thrombus capture success. Apart from these, the retrieval stent 101 that is formed from a mesh tube may not be closed at a trailing end 101a thereof (i.e., a trailing end 101a of the distal curved section 103, as can be seen from Fig. 3a). This enables good compliance and higher resistance to collapse within a tortuous blood vessel, reducing the risk of escape or rupture of a captured thrombus. Additionally, when coming into contact with a blood vessel, the distal curved section 103 will have a smaller contact area therewith, reducing the probability of damage caused to the blood vessel. Further, the above described structural design of the retrieval stent 101 enables it to easily detach a thrombus firmly adhering to the blood vessel and then capture and transport it away, increasing thrombus removal success. Another advantage of the thrombectomy stent(s) 101 is that the outwardly folded and curved structural design can better guide it/them into the delivery device 200, enabling easier withdrawal.

It will also be understood that as a retrieval stent 101 is self-expanding at a lesion site within a blood vessel, the expansion action of the distal curved section 103 can cut and disrupt a large thrombus, which can be more easily aspirated away. Upon full expansion of the retrieval stent 101, the thrombus can be collected in a timely manner within the proximally curved umbrella-shaped mesh surface, thereby capturing the thrombus and reducing the risk of distal escape of thrombus. Additionally, during retraction, the umbrella-shaped mesh surface in the distal curved section 103 of the retrieval stent 101 can tightly contact an inner wall of the blood vessel. Therefore, when retracted, it can effectively "scrape" off and collect any thrombus that strongly adheres to the blood vessel wall. Thus, thrombi in the lumen of a blood vessel and on a wall thereof can be all well removed and captured with high thrombectomy efficiency, resulting in good therapeutic outcomes.

In some embodiments, the device 100 includes multiple retrieval stents 101, which are separately mounted on the core tube 102, with adjacent retrieval stent 101 being arranged at a distance. With this arrangement, one or more thrombi can be further received in space(s) between the umbrella-shaped mesh surfaces of adjacent retrieval stents 101, additionally reducing the risk of distal escape. Further, the presence of the multiple retrieval stents 101 increases the compliance and adaptation of the retrieval device 100 within tortuous blood vessels and blood vessels with a varying diameter and reduces the likeliness of the retrieval stents 101 experiencing inward collapse within such blood vessels, making the retrieval device 100 more powerful in cutting and capturing a thrombus. Furthermore, the multiple retrieval stents 101 entail a multi-segment thrombus removal, which allows for quick removal of a large amount of thrombus from the body.

In the illustrated embodiment, the proximal loading section 105 of each retrieval stent 101 is generally cylindrical (and corresponds to a straight tubular section) and can be entirely disposed over the outer circumference of the core tube 102 around the distal end thereof and then fixed at its proximal end 101b (which provides the proximal end of the retrieval stent 101) to the core tube 102. As exemplified below, the connection between the proximal loading section 105 and the core tube 102 may be accomplished using at least one of various approaches.

Optionally, the proximal loading section 105 may be thermally fused, glued, mechanically attached or otherwise connected to the core tube 102. Here, the mechanical attachment may be accomplished by any method known to those skilled in the art, including, but not limited to, welding. In this embodiment, the proximal loading section 105 is fixed to the core tube 102 with a radiopaque ring 104, which is sleeved over the proximal loading section 105 so that the proximal end 101b of the proximal loading section 105 is fixed to and around the outer circumference of the core tube 102. The radiopaque ring 104 may be made of any suitable radiopaque material, without limitation.

The retrieval stent 101 is a self-expanding mesh structure, which comprises a crimped configuration under external force and will transition from the crimped configuration into an expanded configuration by removing the external force. The expanded configuration is a nature configuration, in which the retrieval stent 101 is not stressed at all. The retrieval stent 101 is loaded and delivered in the delivery device 200 and comprises a crimped configuration. Generally, when in the crimped configuration, the retrieval stent 101 comprises a substantially linear shape extending substantially parallel to the core tube 102. In other words, when the retrieval stent 101 is crimped, the distal curved section 103 is flipped so as to be substantially collinear with the proximal loading section 105. This enables a reduced size during delivery. After released from the delivery device 200, the retrieval stent 101 can expand to an expanded configuration. The retrieval stent 101 is switchable between the crimped and expanded configurations.

The retrieval stent 101 is usually made of a metallic material, such as a metal or alloy. A metallic material with shape memory properties is particularly preferred, examples of which may include, but are not limited to, nickel-titanium alloys. Metal mesh tubes may be prepared using various methods known in the art and then subjected to a shape setting process, forming the retrieval stents 101 having a proximal loading section 105 and a distal curved section 103 integral therewith. As well known in the field of heat setting, molds can be used to maintain the metal mesh tubes in a desired final configuration, and appropriate heat treatment may be then applied to set the retrieval stents 101 into the desired configuration.

In some implementations, the retrieval stent 101 is integrally formed by cutting a metal tube. Suitable cutting methods may include, but are not limited to, laser cutting. In other implementations, the retrieval stent 101 is integrally braided with multiple metal wires. The present invention is not limited to any particular braiding method.

The number of the retrieval stents 101 may depend on, amongst others, the length of a target lesion (e.g., a thrombus) and a desired thrombectomy length for the retrieval stents 101. For example, in the case of thrombus removal, in order to quickly remove a large amount of thrombus, multiple retrieval stents 101 are usually arranged to remove thrombus from the body in segments. When there are only a small number of thrombi to be removed, only one retrieval stent 101 may be arranged. The multiple retrieval stents 101 may have equal or different lengths and radial dimensions. It would be appreciated that the use of multiple retrieval stents 101 enables a thrombus to be disrupted by multiple times, providing better thrombus disruption. In addition, this can also increase the compliance and adaptation of the device 100 within tortuous blood vessels and blood vessels with a varying diameter.

The present invention is not limited to any particular shape of mesh openings 107 in the retrieval stents 101, and the mesh openings 107 may have any appropriate shape, such as rhombic, diamond-like, rectangular, circular or elliptical. Other shapes are also possible. In some implementations, the proximal loading sections 105 have substantially rhombic or diamond-like mesh openings 107, while the distal curved sections 103 may have mesh openings of any suitable shape. Possible examples of the shape of the mesh openings 107 in the distal curved sections 103 may include rectangular, circular, rhombic, elliptical, or a combination thereof.

In this embodiment, the proximal loading section 105 has a substantially constant density of mesh openings, while each distal curved section 103 has a density of mesh openings which varies as the distal curved section 103 is outwardly folded and curved, the mesh opening density of distal curved section 103 decreases from the center to the periphery, thus the distal curved section 103 forms an mesh opening-dense region 1031 close to the center and an mesh opening-sparse region 1032 away from the center, as shown in Fig. 3c. The mesh opening-dense region 1031 has more mesh openings than the mesh opening-sparse region 1032, but the mesh openings in the mesh opening-dense region 1031 are smaller (in diameter) than those in the mesh opening-sparse region 1032. The mesh opening-dense region 1031 has better thrombus collection performance and is associated with a reduced probability of distal thrombus escape. In contrast, the mesh opening-sparse region 1032 is more powerful in disrupting large thrombus during outward folding, expansion and curving of the retrieval stent 101, or during retraction thereof. This density profile with denser mesh openings around the center and sparser mesh openings along the periphery enables both good thrombus retention and reduced resistance to sheathing.

For the retrieval stent 101, in the fully expanded configuration thereof, a radial dimension of the distal curved section 103 at a certain location (e.g., where a maximum diameter thereof is defined) may be optionally greater than or equal to a diameter of a blood vessel at a target lesion site. This allows the outwardly folded distal curved section 103 to tightly contact and support an inner wall of the blood vessel while being movable on the inner wall. Thus, during retraction, it can effectively "scrape" off and collect any adherent thrombus on the inner wall of the blood vessel, and can collect disrupted thrombus within the blood vessel into its umbrella-shaped mesh surface, while allowing blood to flow therethrough. Ensuring smooth blood flow is meaningful because the patient's life may be otherwise threatened.

The core tube 102 is an elongate tube, and a guide wire 10 can be passed into the interior thereof. In the illustrated embodiment, a guide cap 106 is fixed to the core tube 102 at a distal end thereof. A distal portion of the guide cap 106 has a smooth surface, which causes almost no damage to surrounding tissue. The guide cap 106 is provided to facilitate advancement of the retrieval device 100 within a lumen. The guide cap 106 may be a hollow cone with a spherical distal end, and there may be a step at the junction of the cone and the core tube 102. The step may be brought into abutment with a distal end face of a delivery catheter 201 in the delivery device 200 and thereby blocked from entering the interior of the delivery catheter 201. In practice, the core tube 102 may be manipulated to release the retrieval device 100 by pushing it out of the delivery device 200. If the retrieval device 100 is released at a wrong location, it may be retracted back into the delivery device 200 and then again released after location adjustment. After thrombectomy, the core tube 102 may be again manipulated to retract the retrieval device 100 back into the delivery device 200, thereby withdrawing the thrombus out of body.

In one implementation, the core tube 102 further includes an elastic section and al stiff section. The elastic section is arranged at the distal end of the core tube 102 and the stiff section is arranged at the proximal end of the core tube 102. The elastic section is softer than the stiff section and configured for mounting of all the retrieval stents 10 thereon. The elastic and stiff sections may be integrally formed, or separately formed and then assembled together, for example by coupling them to each other. A distal end of the stiff section may be coupled to a proximal end of the elastic section by heat welding, snap engagement or another known technique, and the present invention is not limited to any particular method for coupling. The stiff section of the core tube 102 is more capable of force transfer, and the elastic section of the core tube 102 is freely stretchable, retractable and bendable in response to bending of the retrieval stents 101, increasing the compliance and adaptation of the device 100 in tortuous blood vessels and blood vessels with a varying diameter. In other implementations, the entire core tube 102 may be stiff section.

In some embodiments, the delivery device 200 includes an inner delivery catheter 201 and an outer sheath 202 arranged coaxially therewith. The core tube 102 passes through the delivery catheter 201. The delivery catheter 201 is able to crimp and deliver all the retrieval stents 101 to a site in need of thrombectomy. The delivery catheter 201 further needs to extend through the sheath 202 and to be advanced therein. After thrombectomy, the retrieval device 100 can be guided and retracted back into the sheath 202. The proximal end of sheath 202 is sealed with a sealing valve to prevent leakage of blood from the proximal end of the sheath 202.

In some embodiments, the aspiration device 300 includes an aspiration tube 301 and a coupling base 302. The aspiration device 300 may also include an aspiration device (not shown). The coupling base 302 is detachably disposed at a proximal end of the delivery catheter 201, and a flexible sealing valve 303 for preventing blood leakage is often arranged at an open proximal end of the coupling base 302 to sealingly close the proximal end of the coupling base 302. When a proximal end of the delivery catheter 201 is coupled to coupling base 302 to form a main passage, through which the retrieval device 100 can be moved into and out of the delivery catheter 201. The aspiration tube 301 is disposed on one side of the coupling base 302, and the distal end of the aspiration tube 301 is connected to a lateral hole of the coupling base 302. The proximal end of aspiration tube 301 is often provided with a one-way valve 304 configured for connection with the aspiration device. The aspiration device is able to apply a suction force to the delivery catheter 201 through the aspiration tube 301. The aspiration device may be selected from negative pressure aspiration device, such as a vacuum pump.

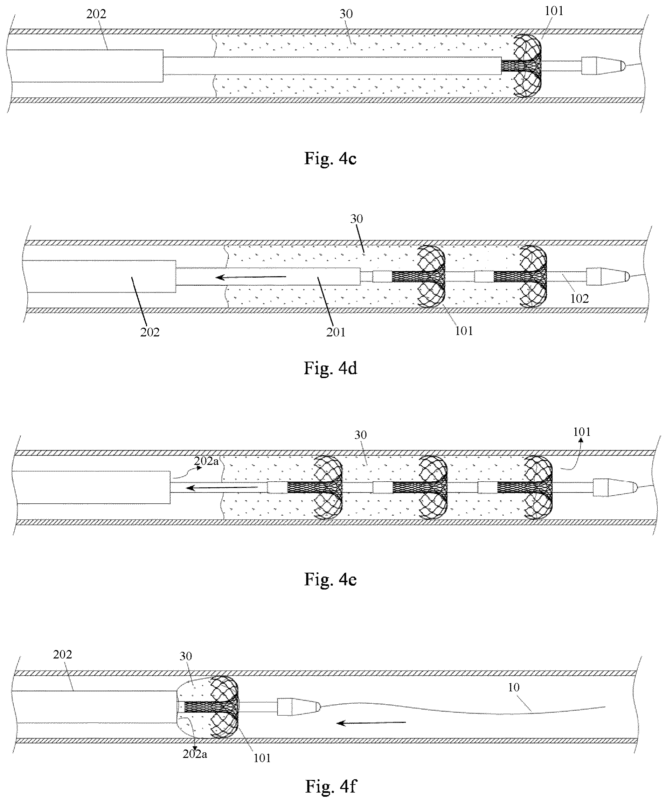

Next, a process to use the retrieval system 100 is described with reference to Figs. 4a to 4f in the context of removing a thrombus from a blood vessel as an example.

As shown in Fig. 4a, in order to remove the thrombus from a diseased site inside the blood vessel, a guide wire 10 is first advanced through the blood vessel 20 to the thrombus 30 and then further pushed to completely pass through the entire thrombus 30.

As shown in Fig. 4b, the sheath 202 is advanced over the guide wire 10 to a location near the thrombus 30 and then held still there, and the delivery catheter 201 with the retrieval device 100 being loaded therein is then advanced also over the guide wire 10 until the guide cap 106 at the distal end of the core tube 102 completely passes through the thrombus 30.

Subsequently, as shown in Figs. 4c to 4e, with the core tube 102 being held stationary, the delivery catheter 201 is gradually retracted until the retrieval stent 101 fully expands, at this point, the distal curved section 103 of the retrieval stent 101 adequately penetrates into and disrupts the thrombus 30. The aspiration device is then activated to suck the thrombus at a distal opening 202a of the sheath 202 into the delivery catheter 201 and into the aspiration tube 301. In the case of multiple retrieval stents 101, they will successively expand during retraction of the delivery catheter 201.

As shown in Fig. 4f, after all the retrieval stents 101 expand, the core tube 102, and hence the retrieval stents 101, is slowly retracted to proximal end, thereby gradually peeling off, squeeze, cutting and disrupting the thrombus 30, with the latter being trapped between the distal curved sections 103 of adjacent retrieval stents 101, and gradually displaced toward the distal opening 202a of the sheath 202. In this process, the aspiration device is kept to continually aspirate the thrombus 30 until the last retrieval stent 101 is retracted into the sheath 202. At this point, the thrombus will be partially collected in the sheath 202. In this way, the thrombus will be eventually extracted out of the body together with the retrieval device 100.

Optionally, the mesh opening densities of the multiple retrieval stents 101 in the retrieval device 100 gradually decrease from the distal end to the proximal end. Thus, a more proximal retrieval stent 101 has sparse mesh openings 107, which facilitate further trap and disruption of large thrombus. Moreover, a more distal retrieval stent 101 has denser mesh openings 107, which help trap finer thrombus and prevent their distal escape. However, in other implementations, the retrieval stents 101 may be consistent in mesh opening density.

### <Embodiment 2>

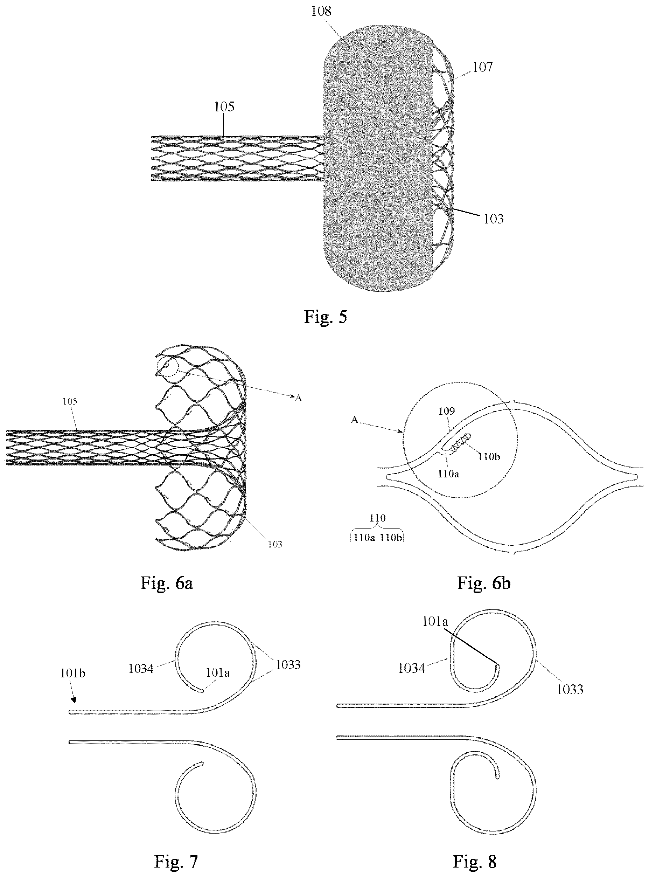

Referring to Fig. 5, a retrieval stent 101 according to a second embodiment of the present invention is substantially similar to that of the first embodiment in terms of structure, except for some differences. In the following, only these differences will be described, and the similarities between the two embodiments are not repeated.

As shown in Fig. 5, differing from the first embodiment, the retrieval stent 101 of the second embodiment includes a distal curved section 103 provided with a graft 108, which covers some mesh openings 107 in the distal curved section 103. Optionally, the graft 108 may cover only mesh openings 107 in a mesh opening-sparse region 1032 of the distal curved section 103, but not mesh openings 107 in an mesh opening-dense region 1031 thereof. With the graft 108, the retrieval stent 101 is able to better collect and block disrupted thrombus from escape and thereby provide improved thrombectomy performance, while allowing smooth blood flow in the lumen. The graft 108 may be disposed either on an inner surface or on an outer surface of the distal curved section 103. The graft 108 may be sewn and fixed onto the distal curved section 103.

Further, in case of the number of the retrieval stents 101 being multiple, optionally, at least a distalmost one of the retrieval stents 101 may be provided with a graft 108, while the remaining stent(s) 101 may also be provided with a graft 108, or not.

### <Embodiment 3>

Referring to Figs. 6a and 6b, a retrieval stent 101 according to a third embodiment of the present invention is substantially similar to that of the first embodiment in terms of structure, except for some differences. In the following, only these differences will be described, and the similarities between the two embodiments are not repeated.

As shown in Figs. 6a and 6b, differing from the first embodiment, the retrieval stent 101 of the third embodiment includes a distal curved section 103 provided with a radiopaque structure 110 disposed on or near a mesh strut 109. The radiopaque structure 110 is visible on X-ray images, thereby providing an operator with a basis for observing the location of the retrieval stent 101 within a lumen and its extent of expansion in real time during a surgical procedure. Optionally, the radiopaque structure 110 may be disposed at a location comprising a maximum radial dimension of the distal curved section 103 in an expanded configuration. This allows better observation of adherence of the distal curved section 103 with a luminal wall. Optionally, there are multiple radiopaque structures 110 that are provided on a single circumference. However, those skilled in the art will recognize that the radiopaque structures 110 may be indeed arranged at any appropriate locations on the distal curved section 103, without departing from the scope of the present invention. Additionally, the present invention is not limited to any particular approach for arranging the radiopaque structures 110 on the distal curved section 103, and those skilled in the art can selected at least one of various possible approaches. One example is set forth below.

In one implementation, as shown in Fig. 6b, the radiopaque structure 110 includes an extension strut 110a and a radiopaque object 110b provided on the extension strut 110a. The extension strut 110a is located within a mesh opening 107, with its one end being connected to a mesh strut 109. The other end of the extension strut 110a is a free end. The radiopaque object 110b may be implemented as any of various structures including but not limited to a radiopaque wire and a radiopaque ring. In the illustrated example, the radiopaque object 110b is a radiopaque wire helically wound on the extension strut 110a. The extension strut 110a may be formed as an integral part of a mesh tube, for example, by cutting. This allows for easy fabrication. The radiopaque object 110b may be made of a common radiopaque material, examples of which may include, but are not limited to, a platinum-iridium alloy, tungsten, etc. The radiopaque object 110b may be fixedly attached to the extension strut 110a in various manners, such as welding, gluing or snap engagement. In other implementations, the radiopaque structures 110 may be fabricated by composite wires (e.g., DFT^{®}) that includes a nickel-titanium sleeve and a radiopaque core wire surrounded by the nickel-titanium sleeve.

It should be noted that although this embodiment has been described in the context of each radiopaque structure 110 including an extension strut 110a and a radiopaque object 110b, as will be appreciated by those skilled in the art, the present invention is not so limited. In some alternative embodiments, the radiopaque structures 110 may be implemented as radiopaque sleeves or wires directly disposed over or wound on mesh struts 109. Still alternatively, the radiopaque structures 110 may be provided on intersections of mesh struts, for example, the radiopaque dots may be provided on intersections of mesh struts.

### <Embodiment 4>

Referring to Figs. 7 to 10, a retrieval stent 101 according to a fourth embodiment of the present invention are substantially similar to those of the first embodiment in terms of structure, except for some differences. In the following, only these differences will be described, and the similarities between the two embodiments are not repeated. It should be noted that shown in the figures are simplified schematics of the retrieval stent 101, which show only its contour, but not details of its mesh structure.

Differing from the first embodiment, the retrieval stent 101 of the fourth embodiment includes a distal curved section 103, which may be curved at various different angles into various different shapes.

As shown in Fig. 7, in an exemplary implementation, the distal curved section 103 of the retrieval stent 101 has an inner first mesh surface 1033 and an outer second mesh surface 1034 directly joined to the first mesh surface 1033. In a fully expanded configuration of the retrieval stent 101, the first mesh surface 1033 is curved toward a proximal end of the retrieval stent 101 and the second mesh surface1034 toward the first mesh surface 1033 from the first mesh surface 1033 so that a trailing end 101a of the retrieval stent 101 is located within an umbrella-shaped mesh surface and is oriented toward the first mesh surface 1033. With this arrangement, when the retrieval stent is being retracted, a considerable proportion of resistance to the retraction will be applied to the second mesh surface 1034, lowering the likelihood of the curved mesh surfaces being undesirably flipped distally in the event of a large amount of thrombus and making the distal curved section 103 more resistant to deformation. In the first embodiment, referring to Fig. 3b, the distal curved section 103 of the retrieval stent 101 consists essentially of only the first mesh surface 1033 that curves proximally. In contrast, in the example of Fig. 7, the first mesh surface 1033 comprises a curved mesh and provides a distal block surface, and the second mesh surface 1034 also comprises a curved mesh and provides a proximal block surface, the proximal block surface opposes the distal block surface in a direction of thrombectomy and is closer to the proximal end 101b of the retrieval stent 101 than the distal block surface.

As shown in Fig. 8, in another exemplary implementation, the distal curved section 103 of the retrieval stent 101 is a combination of a curved mesh surface and a polygonal mesh surface. Specifically, the distal curved section 103 consists of an inner first mesh surface 1033 and an outer second mesh surface 1034, which are directly joined to each other. The first mesh surface 1033 is a curved mesh surface, and the second mesh surface 1034 is a polygonal mesh surface. There is a smooth transition connection between the first mesh surface 1033 and the second mesh surface 1034. With this arrangement, similarly, in the fully expanded configuration of the retrieval stent 101, the first mesh surface 1033 is curved toward the proximal end 101b of the stent 101 and the second mesh surface1034 toward the first mesh surface 1033 so that the trailing end 101a of the retrieval stent 101 is within an umbrella-shaped mesh surface and is oriented toward the first mesh surface 1033. In the implementation of Fig. 8, the first mesh surface 1033 provides a distal block surface, and the second mesh surface 1034 provides a proximal block surface. The second mesh surfaces 1034 function primarily to enhance supporting and trapping capabilities of the distal curved section 103 so that the distal curved section 103 has improved supporting and trapping capabilities and thereby impart improved thrombus removal performance to it.

The proximal and distal block surfaces may have the same or different mesh opening densities. Optionally, the proximal block surface may have sparser mesh openings than the distal block surface. With this arrangement, the proximal and distal block surfaces can provide thrombus trapping around proximal and distal ends of the distal curved section 103, respectively. Accordingly, during retraction, larger mesh openings 107 in the proximal block surface can cut and disrupt large thrombus, and denser mesh openings 107 in the distal block surface can trap finer thrombus and block their escape.

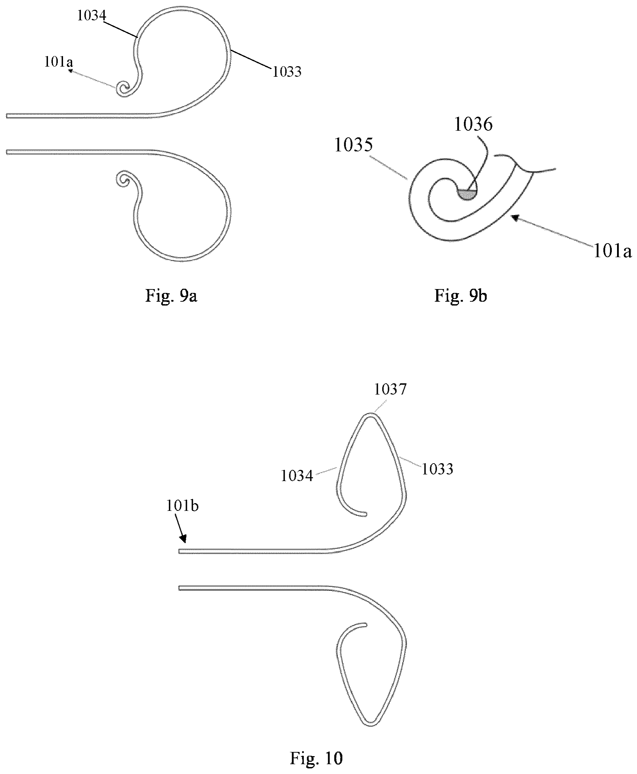

As shown in Figs. 9a and 9b, in yet another exemplary implementation, in the fully expanded configuration of the retrieval stent 101, the distal curved section 103 is folded and curved outwards at the trailing end 101a that is away from a proximal loading section 105, thereby forming a non-invasive end 1035 (as a non-limiting example, in the form of a circular ring). The non-invasive end 1035 may be curved at an angle smaller than, equal to or greater than 360°. The non-invasive end 1036 can reduce possible damage caused by the trailing end 101a to a blood vessel during the self-expansion or retraction of the retrieval stent 101. Optionally, an end face of the non-invasive end 1035 may be covered with a soft covering material 1036. This soft covering material 1036 may be a soft polymer material, which can additionally reduce the risk of damage caused to a blood vessel by the trailing end 101a. With the non-invasive end 1035 and the soft covering material 1036, possible damage caused by the trailing end 101a to a blood vessel can be additionally reduced during the self-expansion of the retrieval stent 101, or during its retraction into the sheath 202.

As shown in Fig. 10, in still yet another exemplary implementation, the distal curved section 103 of the retrieval stent 101 is a combination of curved and polygonal mesh surfaces. Specifically, the distal curved section 103 consists of an inner first mesh surface 1033, an intermediate transition mesh surface 1037 and an outer second mesh surface 1034. The first mesh surface 1033 and the second mesh surface 1034 are both polygonal mesh surfaces, and the transition mesh surface 1037 is a curved mesh surface to provide a smooth transition connection between the transition mesh surface 1037 and the first mesh surface 1033. Correspondingly, a smooth transition connection is provided between the transition mesh surface 1037 and the second mesh surface 1034. Likewise, in the fully expanded configuration of the retrieval stent 101, the first mesh surface 1033 is curved toward the proximal end 101b of the retrieval stent 101, and the second mesh surface 1034 is curved toward the first mesh surface 1033 from the transition mesh surface 1037. In the implementation of Fig. 10, the polygonal first mesh surface 1033 provides a distal block surface, and the polygonal second mesh surface 1034 provides a proximal block surface. Thus, it can be seen that each of such distal and proximal block surface may be provided by either a curved or polygonal mesh surface. In this case, the transition mesh surface 1037 is configured to come into contact with an inner wall of a blood vessel to provide support. This enables the distal curved section 103 to provide desirable support and have a small contact area with a blood vessel, reducing possible damage caused to the blood vessel during thrombus removal.

The above discussed angles of curvature and curved shapes for the distal curved section 103 of the retrieval stent 101 can be obtained by heat setting processes using different molds. A retrieval device 100 according to this embodiment may include retrieval stents 101 of one or more of the above types. The present invention is not limited to any of the angles of curvature and curved shapes of the retrieval stent 101 in the above implementations, and those skilled in the art can appropriately design the angle of curvature and curved shape of the retrieval stent 101 as desired in practical applications.

### <Embodiment 5>

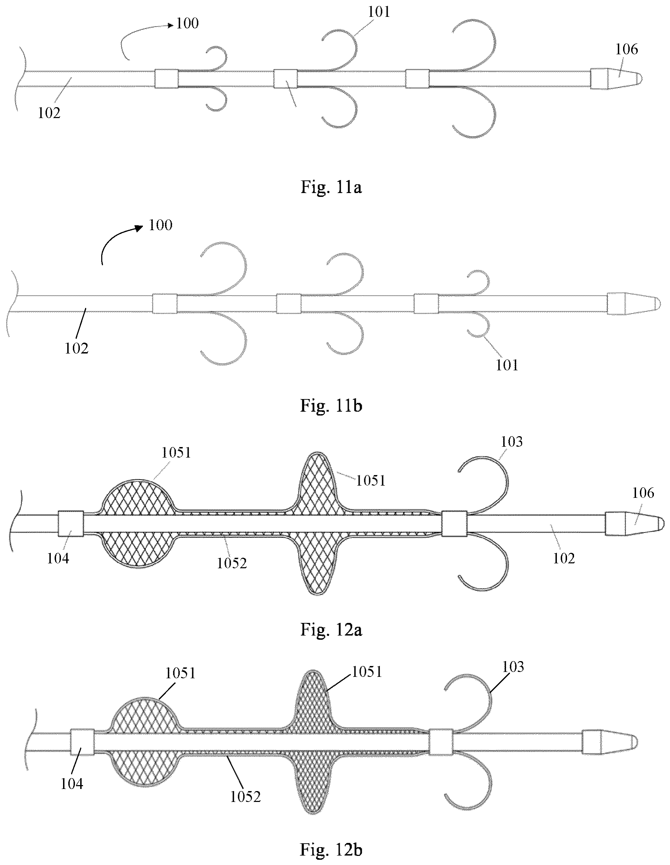

Referring to Figs. 11a to 11b, a retrieval device 100 according to a fifth embodiment of the present invention is substantially similar to that of the first embodiment in terms of structure, except for some differences. It should be noted that Figs. 11a and 11b are a simplified schematic, which show only contours of retrieval stents 101, but not details of their mesh structures.

As shown in Fig. 11a, differing from the first embodiment, in the retrieval device 100 provided in Embodiment 5, a core tube 102 is provided, at a distal end thereof, with a plurality of retrieval stents 101, and the radial dimensions of the plurality of retrieval stents 101 gradually decrease from the distal end to the proximal end. Moreover, the radial dimension of the distalmost retrieval stent 101 may be greater than or equal to a diameter of a blood vessel where the lesion is located. This enables close contact of the distalmost retrieval stent 101 with an inner wall of the blood vessel. It will be understood that the gradually varying radial dimension arrangement helps reduce resistance from thrombus to the retrieval stents 101 at the proximal end during retraction of the device 100. This reduces issues such as excessive deformation or collapse of the proximal retrieval stent 101 caused by excessive thrombus resistance during the retraction. Additionally, constrictive deformation of the more proximal retrieval stents 101 can be well buffered, reducing possible loss of thrombus, during retraction into the delivery device 200. Further, the gradually varying radial dimension of the retrieval stents 101 enables the entire retrieval device 100 to exhibit increased compliance and adaptation in tortuous blood vessels and blood vessels with a varying diameter.

As shown in Fig. 11b, differing from the first embodiment, in the retrieval device 100 provided in Embodiment 5, the core tube 102 is provided, at a distal end thereof, with a plurality of retrieval stents 101, and the radial dimensions of the plurality of retrieval stents 101 gradually increase in the distal end to the distal end. Moreover, the radial dimension of a proximalmost retrieval stent 101 may be greater than or equal to a diameter of a blood vessel where the lesion is located. This enables close contact of the proximalmost stent 101 with an inner wall of the blood vessel. Likewise, this gradually varying radial dimensions of the retrieval stents 101 enable the entire retrieval device 100 to exhibit increased compliance and adaptation in tortuous blood vessels and blood vessels with a varying diameter. In particular, it allows the distal retrieval stent 101 to be inserted into a narrow blood vessel (e.g., a branch vessel in the lungs) to remove thrombus or thrombi therein with reduced likeliness of causing damage to the blood vessel.

### <Embodiment 6>

Referring to Figs. 12a to 12c, a retrieval device 100 according to a sixth embodiment of the present invention is substantially similar to that of the first embodiment in terms of structure, except for some differences. In the following, only these differences will be described, and the similarities between the two embodiments are not repeated.

As shown in Figs. 12a to 12c, differing from the first embodiment, in the retrieval device 100 provided in Embodiment 6, besides the core tube 102, the retrieval device 100 comprises only a single integral retrieval stent 101. This single integral retrieval stent 101 can be formed either by braiding followed by heat-setting or by cutting followed by heat-setting. This integral (or one-piece) retrieval stent 101 includes a distal curved section 103, which is substantially the same as that of any foregoing embodiment, and a proximal loading section 105 is directly configured as multiple bulge units 1051, which are radially raised outwardly. The bulge units 1051 are spaced apart along an axis of the retrieval stent 101 and all integrally formed with the distal curved section 103. The multiple bulge units 1051 may be of the same or different structures, wherein the structure may comprise shape and size. The bulge units 1051 may be connected by straight-extending connecting portion(s) 1052.

In this embodiment, the distal curved section 103 has very dense mesh openings 107 and a diameter in its expanded configuration, which is greater than or equal to a diameter of a blood vessel where the lesion is located. It is configured to collect fine disrupted thrombus, preventing their distal escape.

The bulge unit 1051 may have various appropriate shapes, and the present invention is not limited to any particular shape. Typically, the bulge units 1051 may be in the shape of spheres, discs (or disc-like shapes), cylinders, baskets, bags, or a combination thereof. The bulge units 1051, when having a circular or almost circular cross-section, can provide better support, more effectively remove one or more thrombi within a blood vessel, more effectively scrape off one or more thrombi adhering to a blood vessel wall and reduce the likeliness of the retrieval stents 101 experiencing undesired excessive deformation or collapse due to an excess of thrombus during withdrawal. In addition, when implemented as baskets or bags, the bulge units 1051 can better collect a thrombus or thrombi and block its/their distal escape.

The present invention is not limited to any particular shape, number, diameter in an expanded configuration, or the like of the bulge units 1051, and these may depend upon the particular circumstances of an application or lumen where they are intended to be used.

As shown in Fig. 12a, in one example, the proximal loading section 105 includes a disc-like bulge unit 1051 and a spherical bulge unit 1051. They are not limited to the locations as shown, and may be, for example, swapped in location. The bulge units 1051 are integrally joined by a substantially cylindrical connecting portion 1052. When the retrieval stent 101 is in a fully expanded configuration, a proximal bulge unit 1051 may optionally have a radial dimension smaller than a radial dimension of the other bulge units 1051. Of course, it is contemplated herein that each bulge unit 1051 may have an equal radial dimension. Each bulge unit 1051 may expand to a diameter equal to or greater than a diameter of a target blood vessel. In addition, when expanded, radial dimensions of the bulge units 1051 may gradually decrease or increase in a distal-to-proximal direction.

As shown in Fig. 12b, in another example, mesh opening densities of the bulge units 1051 gradually decrease in the distal-to-proximal direction. The proximal sparser mesh openings enable better disruption of large thrombus, and the distal denser mesh openings can reduce the likeliness of distal thrombus escape.

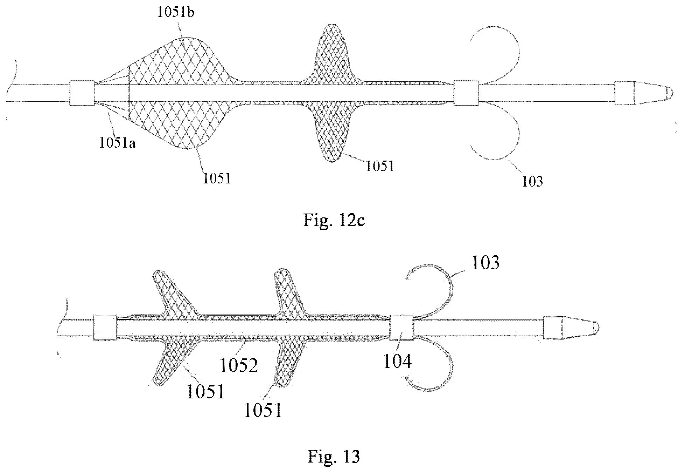

As shown in Fig. 12c, in yet another example, the proximal loading section 105 includes a disc-like bulge unit 1051 and a basket-shaped bulge unit 1051. They are not limited to the locations as shown, and may be, for example, swapped in location. In this implementation, the basket-shaped bulge unit 151 includes projecting struts 1051a and a bag 1051b. When retracted, the projecting struts 1051a can cut large thrombus, which are then collected in the interior of the bag 1051b. This design combines the advantages of the various types of bulge units 1051 and provides improved thrombectomy performance.

Referring to Figs. 12a to 12c, the retrieval stent 101 is fixed at both its proximal and distal ends to the core tube 102. Specifically, the proximal end of the most proximal bulge unit 1051 is fixed to the core tube 102 to form a proximal fixation point, and a transitional connection of the most distal bulge unit 1051 and the distal curved section 103 is fixed to the core tube 102 to form a distal fixation point. The proximal loading section 105 is substantially disconnected from the core tube 103 between the proximal and distal fixation points to facilitate smooth blood flow. In the illustrated example, the proximal end of the most proximal bulge unit 1051 is fixed with a radiopaque ring 104, and a transitional connection of the most distal bulge unit 1051 and the distal curved section 103 is fixed using another radiopaque ring 104.

It should be noted that there may be one or more bulge units 1051, where more is not limited to two.

### <Embodiment 7>

Referring to Fig. 13, a retrieval device 100 according to a seventh embodiment of the present invention is substantially similar to that of any of the foregoing embodiments, except for some differences. In the following, only these differences will be described, and the similarities between the two embodiments are not repeated.

As shown in Fig. 13, different from the foregoing embodiments, the retrieval device 100 of this embodiment is suited to use for removal of a stone from a bile duct or ureter. The removal is accomplished in the substantially same manner as described above for thrombectomy.

For example, the retrieval device 100 may include an integral retrieval stent 101 including bulge units 1051. In other examples, a proximal loading section 105 includes multiple bulge units 1051 each in the shape of a disc. All these bulge units 1051 can be distally or proximally deflected, thereby reducing resistance during retraction of the bulge units 1051 into the sheath tube 202.

In the illustrated example, two disc-like bulge units 1051 deflects proximally, and the bulge units 1051 are connected by a substantially cylindrical connecting portion 1052. The bulge units 1051 have dense mesh openings.

When in a fully expanded configuration of the retrieval stent 101, the bulge units 1051 and a distal curved section 103 of the retrieval stent 101 will all come into contact with an inner wall of a lumen around a stone. Thus, during a surgical procedure, the retrieval stent 101 may be moved within the lumen to carry the stone out thereof. The arrangement of the multiple bulge units 1051 form a layered stone removal structure which can take repeated removal actions within the lumen, avoid any stone from remaining in the lumen and resulting in improved stone removal efficiency. Moreover, the dense mesh openings in the bulge units 1051 can effectively block the passage of small stone therethrough. Further, in the stone removal process, an external aspiration device may be used to suck distal small stone into an aspiration tube 301, increasing complete stone removal success.

As described above, the proposed retrieval stent, retrieval device and retrieval system offer at least the following benefits:
First, the distal opening of the retrieval stent is outwardly curved to generally form an umbrella-like shape. This structure allows the retrieval stent to have a small contact area with a lumen, which can reduce damage to a wall of the lumen during removal of a thrombus, stone or other occlusive object from the lumen. In addition, it can facilitate retraction of the retrieval stent back into a delivery device during withdrawal.
Second, the device may include multiple such retrieval stents, or the retrieval stent itself may be configured to comprise multiple bulge units. This multi-segment design imparts to the retrieval device increased compliance and adaptation within a tortuous blood vessel or a blood vessel with a varying diameter, which reduce the likelihood of the stent experiencing inward collapse within a tortuous lumen, enhance its ability to cut and capture an occlusive object, and facilitate its delivery and withdrawal.
Third, after the retrieval device fully expands, the outwardly folded mesh surface of a retrieval stent will come into close contact with an inner wall of the lumen. Thus, when the device is retracted, the occlusive object that adheres to the luminal wall can be effectively removed, achieving better occlusive object removal.
Fourth, when in the fully expanded configuration of the retrieval device, occlusive can be collected in umbrella-shaped mesh surface(es). In particular, more occlusive object can be received within space(s) between adjacent retrieval stents or bulge units. Thus, occlusive object removal can be achieved with higher efficiency.
Fifth, the mesh opening densities of multiple stents or bulge units gradually decrease in a distal-to-proximal direction. With this arrangement, the distal denser mesh openings can collect an occlusive object with a reduced risk of distal escape thereof, and the proximal sparser mesh openings enable the retrieval device, when retracted, to disrupt a large occlusive object while transporting and delivering the occlusive object into an aspiration tube. In this way, good removal performance and high removal efficiency can be obtained.

Further, it will be recognized that while the invention has been described above with reference to preferred embodiments thereof, it is not intended to be limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. A retrieval stent that is self-expandable, wherein the retrieval stent is fabricated from a mesh tube and comprising a distal curved section and a proximal loading section, wherein a portion of the mesh tube is folded toward an exterior of the mesh stent to form the distal curved section , wherein a non-folded portion of the mesh tube forms the proximal loading section, and wherein the distal curved section comprises an umbrella-shaped mesh surface that is concave toward a proximal end of the retrieval stent.

2. The retrieval stent according to claim 1, wherein a radial dimension of the distal curved section is greater than or equal to a diameter of a target lumen, and wherein a mesh opening density of the distal curved section gradually decreases from a center to a periphery.

3. The retrieval stent according to claim 2, further comprising a graft provided over the distal curved section, wherein the graft covers a portion of the mesh openings in the distal curved section.

4. The retrieval stent according to claim 3, wherein the graft is provided over a mesh opening-sparse region of the distal curved section.

5. The retrieval stent according to claim 1, wherein a radiopaque structure is provided on the distal curved section, and wherein the radiopaque structure is provided at a location of the distal curved section with a maximum radial dimension.

6. The retrieval stent according to claim 5, wherein the radiopaque structure comprises an extension strut and a radiopaque object provided on the extension strut, wherein the extension strut is located within a mesh opening of the distal curved section, wherein one end of the extension strut is connected to a mesh strut, and wherein the other end of the extension strut is a free end.

7. The retrieval stent according to claim 1, wherein the distal curved section comprises a proximal block surface and a distal block surface, wherein the proximal block surface and the distal block surface oppose each other along an axis of the retrieval stent, and wherein the proximal block surface is closer to the proximal end of the retrieval stent than the distal block surface.

8. The retrieval stent according to claim 7, wherein the distal block surface is curved toward the proximal end of the retrieval stent, wherein the proximal block surface is curved toward the distal block surface, and wherein the proximal block surface comprises sparser mesh openings than the distal block surface.

9. The retrieval stent according to claim 7, wherein the proximal block surface comprises a curved shape or a polygonal shape, and wherein the distal block surface comprises a curved shape or a polygonal shape.

10. The retrieval stent according to claim 9, wherein the proximal and distal block surfaces comprise a curved shape and are directly joined to each other, or wherein the distal block surface comprises a curved shape and the proximal block surface comprises a polygonal shape, that are directly joined to each other, or wherein the proximal and distal block surfaces comprise a polygonal shape and are connected by a curved transition mesh surface.

11. The retrieval stent according to claim 1, the distal curved section is outwardly folded and curved at a trailing end thereof that is away from the proximal loading section, thereby forming a non-invasive end, wherein a soft covering material is applied to an end face of the non-invasive end.

12. The retrieval stent according to claim 1, wherein the proximal loading section is a straight tubular section, or wherein the proximal loading section comprises a plurality of bulge units that are radially raised outwardly, wherein the plurality of bulge units are arranged at an interval along an axis of the retrieval stent, and wherein adjacent bulge units are connected by a straight-extending connecting portion, and wherein the plurality of bulge units is integrally formed with the distal curved section.

13. The retrieval stent according to claim 12, wherein structures of the plurality of bulge units are same or different, and wherein the bulge unit comprises a shape of a sphere, a disc, a cylinder, a basket, a bag or a combination thereof.

14. The retrieval stent according to claim 12 or 13, wherein radial dimensions of the plurality of bulge units gradually increase or decrease from a distal end to the proximal end of the retrieval stent, and/or wherein mesh opening densities of the plurality of bulge units gradually decrease from the distal end to the proximal end of the retrieval stent.

15. The retrieval stent according to claim 12 or 13, wherein the bulge unit is proximally or distally deflectable.

16. A retrieval device, comprising a core tube and the retrieval stent of any one of claims 1 to 15, wherein the retrieval stent is disposed over an outer circumference of the core tube at a distal end thereof, and wherein the retrieval stent is attached to the core tube though the proximal loading section.

17. The retrieval device according to claim 16, comprising a plurality of retrieval stents, and wherein the plurality of retrieval stents are arranged at an interval along an axis of the core tube.

18. The retrieval device according to claim 17, wherein mesh opening densities of the plurality of retrieval stents gradually decrease from the distal end to a proximal end of the core tube, and/or wherein radial dimensions of the plurality of retrieval stents gradually decrease or increase from the distal end to the proximal end of the core tube.

19. The retrieval device according to claim 17 or 18, wherein a graft is provided on the distal curved section, wherein the graft covers a portion of the mesh openings of the distal curved section, and wherein the graft is at least provided at a distalmost retrieval stent.

20. The retrieval device according to claim 16, wherein a proximal end of the proximal loading section is fixed to the core tube, or wherein each of the proximal end and a distal end of the proximal loading section is fixed to the core tube.

21. The retrieval device according to claim 20, wherein the proximal loading section is fixed to the core tube by a radiopaque ring.

22. The retrieval device according to claim 20, comprising a single retrieval stent, wherein the proximal loading section comprises a plurality of bulge units that are radially raised outwardly, wherein the plurality of bulge units are arranged at an interval along an axis of the retrieval stent, wherein the plurality of bulge units are integrally formed with the distal curved section, wherein a proximalmost bulge unit is fixed at a proximal end to the core tube and a proximal fixation point is formed, wherein a transitional connection between the distal curved section and a distalmost bulge unit is fixedly connected to the core tube and a distal fixation point is formed, and wherein a portion of the proximal loading section that is located between the proximal fixation point and the distal fixation point is disconnected from the core tube.

23. The retrieval device according to claim 16, wherein the core tube comprises a proximal stiff section and a distal elastic section, wherein the retrieval stent is disposed over the elastic section.

24. A retrieval system, comprising:
the retrieval device of any one of claims 16 to 23;
a delivery device configured to load and deliver the retrieval device to a target lesion site; and
an aspiration device configured for connection with the delivery device and configured to apply a suction force through the delivery device to aspirate an occlusive object from the target lesion site.
